(19) Europäisches Patentamt
European Patent Office
Office européen des brevets

(11) Veröffentlichungsnummer : **0 101 836**
**B1**

(12) # EUROPÄISCHE PATENTSCHRIFT

(45) Veröffentlichungstag der Patentschrift : **22.01.86**

(51) Int. Cl.[4] : **C 07 C121/84**

(21) Anmeldenummer : **83106501.6**

(22) Anmeldetag : **04.07.83**

(54) **Verfahren zur Herstellung von reinem Diacetonitril.**

(30) Priorität : **20.08.82 DE 3231052**

(43) Veröffentlichungstag der Anmeldung : **07.03.84 Patentblatt 84/10**

(45) Bekanntmachung des Hinweises auf die Patenterteilung : **22.01.86 Patentblatt 86/04**

(84) Benannte Vertragsstaaten : **AT BE CH DE FR GB IT LI NL**

(56) Entgegenhaltungen : **GB-A- 993 300**

(73) Patentinhaber : **CHEMIE LINZ AKTIENGESELLSCHAFT
St. Peter-Strasse 25
A-4020 Linz (AT)
BE CH FR GB IT LI NL AT
Lentia Gesellschaft mit beschränkter Haftung
Arabellastrasse 4 Postfach 81 05 08
D-8000 München 81 (DE)
DE**

(72) Erfinder : **Stern, Gerhard, Dipl. Ing. Dr.
Lustenauerstrasse 17
A-4020 Linz (AT)**
Erfinder : **Grossgut, Franz
Hertzstrasse 27
A-4020 Linz (AT)**
Erfinder : **Joos, Michael
Schrammlstrasse 31
A-4050 Traun (AT)**
Erfinder : **Weismann, Helmut
Pulvermühlstrasse 11
A-4040 Linz (AT)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 101 836 B1

Jouve, 18, rue St-Denis, 75001 Paris, France

**Beschreibung**

Die Erfindung betrifft ein Verfahren zur Herstellung von reinem Diacetonitril aus Acetonitril und geschmolzenem Natrium.

Diacetonitril, oder mit anderem Namen 3-Amino-crotonitril, kann nach einer bekannten 2-Stufen-Reaktion aus Acetonitril und Natrium und anschließendem Zersetzen des gebildeten Natriumsalzes hergestellt werden. Ohne auf die möglichen cis-trans isomeren und tautomeren Formen einzugehen, kann die Reaktion durch folgende Reaktionsgleichungen beschrieben werden:

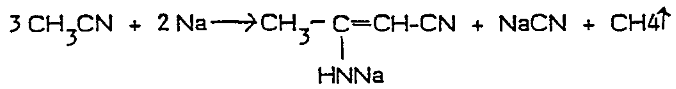

1.

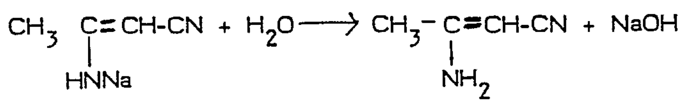

2.

Nach Homer Adkins, Am. Soc. *64,* pg 150-154 (1942) erfolgt die Umsetzung von Acetonitril und Natrium unter Kühlen von außen in Benzol unter Rückflußbedingungen, die gebildeten Salze, nämlich Natriumcyanid und Natriumaminocrotonitril werden abfiltriert, in Äther suspendiert und langsam mit Wasser zersetzt. Aus der abgetrennten ätherischen Lösung kristallisiert rohes Diacetonitril in 65 %iger Ausbeute, das anschließend aus Benzol umkristallisiert wird.

Nach CH-PS 415 603 ist es nachteilig, unter Rückflußbedingungen zu arbeiten, daher erfolgt die Kondensation von Acetonitril und Natrium in einem relativ hoch siedenden aliphatischen Kohlenwasserstoff bei einer Temperatur von 10-35 °C. Zur Hydrolyse des Natriumsalzes wird langsam soviel Wasser zugegeben, daß die Temperatur 40 °C nicht überschreitet. Dabei entstehen im Reaktor drei Schichten, eine obere aus Kohlenwasserstoff, eine mittlere aus Diacetonitril und Acetonitril und eine untere aus wäßrigem Natriumcyanid und Natriumhydroxid, worauf das Diacetonitril aus der mittleren Schicht gewonnen wird. Die Art der Gewinnung ist nicht angegeben, das Diacetonitril hat eine Reinheit von 95-96 %.

Die Hauptschwierigkeiten in beiden Verfahren sind in der Verwendung von festem Natrium zur stark exothermen Kondensationsreaktion begründet. Festes Natrium bildet an seiner Oberfläche gemäß Reaktionsgleichung (1) sogleich das Natriumsalz von Diacetonitril. Da dieses anorganische Salz in dem umgebenden organischen Medium unlöslich ist, bildet sich auf der Metalloberfläche eine undurchlässige, gut haftende Kruste und die Weiterreaktion des metallischen Natriums ist weitgehend unterbunden. Dementsprechend beträgt die Reaktionszeit allein für die erste Stufe sowohl im 1 l-Maßstab bei Adkins, als auch im 100 l-Maßstab in CH-PS 415 603 zwei bis zweieinhalb Stunden, die Raum-Zeit-Ausbeute ist daher äußerst schlecht und wirtschaftlich nachteilig.

Auch die Schwierigkeiten der zweiten Reaktionsstufe, Zersetzung des Natriumsalzes zu freiem Diacetonitril sind in der Salzkruste, im Vorhandensein kleiner, nicht umgesetzter Natriumteilchen bedingt, weshalb die Zugabe von Wasser zur Zersetzung der Natriumsalze unter Vorsichtsmaßnahmen und sehr langsam, in CH-PS 415 603 etwa in einem Zeitraum von 2 Stunden, erfolgen muß.

Vor allem ist es jedoch nach den bisher bekannten Verfahren nicht möglich, hochreines Diacetonitril ohne mehrfaches Umkristallisieren zu erhalten, nach CH-PS 415 603 etwa entsteht ein 95%iges Produkt, nach Adkins ein Rohprodukt. Solche Reinheitsgrade sind jedoch für Diacetonitril, das etwa zur Herstellung pharmazeutischer Wirkstoffe verwendet wird, völlig ungeeignet, und zusätzlich aufwendige Reinigungsschritte sind notwendig.

Überraschenderweise wurde nun ein Verfahren zur Herstellung von Diacetonitril gefunden, das kurze Reaktionszeiten aufweist, bei dem keine Gefahr des Übrigbleibens von nicht umgesetztem Natrium besteht, und bei dem das Diacetonitril ohne Umkristallisieren in einer Reinheit von über 99,5 % entsteht. Dies gelingt, indem die Umsetzung bei einer Temperatur erfolgt, die über dem Schmelzpunkt von Natrium liegt, und indem das nach Hydrolyse des Natriumsalzes und Abtrennung der wäßrigen und der Lösungsmittelphase gebildete flüssige Diacetonitril in kaltem Wasser ausgefällt wird.

Gegenstand der Erfindung ist demnach ein Verfahren zur Herstellung von reinem Diacetonitril durch Umsetzen von Acetonitril mit Natrium in einem inerten, unter Reaktionsbedingungen flüssigen organischen Medium, anschließende Hydrolyse des in erster Stufe gebildeten Natriumsalzes und Gewinnung des Diacetonitrils aus dem dabei entstehenden 3-Phasengemisch, das dadurch gekennzeichnet, ist, daß die Umsetzung mit geschmolzenem Natrium bei einer Temperatur von 97 bis 140 °C und unter einem Druck von 1 bis 10 bar durchgeführt wird, aus dem nach der Hydrolyse gebildeten Dreiphasensystem die wäßrige Phase und das inerte flüssige Medium abgetrennt werden, die dritte Phase aus rohem flüssigem Diacetonitril mit der 0,5- bis 3 fachen Gewichtsmenge Wasser, bezogen auf Gewicht Diacetonitril, versetzt und auf − 10 °C bis + 30 °C abgekühlt wird, worauf das reine Diacetonitril auskristallisiert und abgetrennt wird.

Der erste Reaktionsschritt, Bildung des Natriumsalzes von Diacetonitril erfolgt, indem Natrium in

einem vorher mit Stickstoff gespülten, beheizbaren und kühlbaren Rührbehälter mit Rückflußkühler in Gegenwart eines gegenüber Natrium inerten flüssigen Mediums geschmolzen wird, und unter ständigem intensiven Rühren Acetonitril zugeführt wird. Das inerte flüssige Medium soll zweckmäßig einen Siedepunkt von mindestens 97 °C, dem Schmelzpunkt von Natrium, bis zu etwa 200 °C aufweisen, üblicherweise genügt ein Siedepunkt bis etwa 140 °C. Beispiele für solche inerte flüssige Medien sind aliphatische, cycloaliphatische oder aromatische Kohlenwasserstoffe, beispielsweise Benzine mit dem entsprechenden Siedebereich, Dekaline, Toluol oder Gemische dieser Lösungsmittel.

Die Umsetzung erfolgt bei einer Temperatur von 97-140 °C, vorzugsweise bei 100-120 °C, wobei der Temperaturbereich bis 110 °C besonders bevorzugt ist. Die Reaktion wird bei Atmosphärendruck oder insbesondere bei Verwendung von Kohlenwasserstoffen mit relativ niedrigen Siedepunkten bei erhöhten Drücken, die den Dampfdrücken der Lösungsmittel bei der gewählten Reaktionstemperatur entsprechen, durchgeführt. Der Druck beträgt 1-10 bar, vorzugsweise 2-5 bar.

Das eingetragene Acetonitril reagiert praktisch augenblicklich, sodaß die verdampfende, im Rückflußkühler zu kondensierende Menge gering ist. Zweckmäßig ist es, das Acetonitril unter die Oberfläche des Reaktionsgemisches, bevorzugt am Boden des Reaktionsgefäßes, einzuführen.

Die Geschwindigkeit der Reaktion ist unter den genannten Bedingungen außerordentlich hoch und in der Praxis nur durch die Abführung der Reaktionswärme und damit nur vom Reaktortyp her begrenzt. Das gemäß der Reaktionsgleichung entstehende Methan wird kontinuierlich abgeblasen.

Da das Natrium bei der Reaktion vollständig umgesetzt wird, wird auch nur die theoretisch nötige Acetonitrilmenge für den Umsatz benötigt. Je nach Reaktionsführung kondensieren geringe Acetonitrilmengen im Kühler und werden der Reaktion wieder zugeführt, bis die theoretische Menge Acetonitril verbraucht ist. Es ist auch möglich, die Reaktion mit einem geringen, etwa 10 %igen Überschuß durchzuführen, und nach Beendigung der Reaktion, da die Temperatur der Reaktionsmischung ohnehin wesentlich über dem Siedepunkt des Acetonitrils liegt, das Acetonitril über den Kühler abzudestillieren. Werden Lösungsmittel verwendet, deren Siedepunkt nicht wesentlich über jenem des Acetonitrils liegt, können auch Anteile dieses Lösungsmittels mit abdestilliert werden. In jedem Fall ist das Destillat für den nächsten Ansatz verwendbar.

Die Suspension wird abgekühlt und bei Temperaturen von 0-50 °C, vorzugsweise bei 15-35 °C mit soviel Wasser versetzt, daß das Natriumsalz des Diacetonitrils zu freiem Diacetonitril hydrolysiert wird, und das dabei anfallende Natriumhydroxid sowie das im ersten Reaktionsschritt gebildete Natriumcyanid in eine wäßrige Lösung übergeführt werden. Die Wassermenge wird daher so gewählt, daß sie ausreicht, um alles Natriumsalz zu zersetzen und alles Natriumhydroxid und Natriumcyanid vollständig in Lösung zu bringen. Pro Gewichtsteil eingesetztes Acetonitril benötigt man etwa 1-1,5 Gewichtsteile Wasser.

Da Diacetonitril zu einem geringen Teil in Wasser löslich ist, wird zum Zersetzen des Natriumsalzes bevorzugt die im späteren Kristallisationsschritt anfallende Zentrifugenmutterlauge verwendet. Da die Reaktion praktisch ohne störende Nebenreaktionen vor sich geht, muß nur ein geringer Teil derselben zwecks Entfernung von Nebenprodukten ausgeschleust werden.

Da das Natrium bei Einhaltung der erfindungsgemäßen Vorgangsweise restlos verbraucht wird, kann das Wasser rasch und ohne besondere Sicherheitsmaßnahmen zugeführt werden, wobei als zeitbestimmender Faktor nur die zur Einhaltung der oben genannten Temperaturobergrenze nötige Abführung der Reaktions- bzw. Lösungswärme anzusehen ist.

Nach der Zersetzung liegen drei Phasen vor. Die oberste besteht aus dem inerten organischen Lösungsmittel, sie wird abgetrennt und kann immer wieder in die erste Stufe rückgeführt werden, da sie keine Nebenprodukte enthält. Die unterste Schicht, eine wäßrige Lösung aus Natronlauge und Natriumcyanid wird abgetrennt. Die mittlere Schicht, bestehend aus flüssigem Diacetonitril, etwas Wasser und geringen Mengen gelöster Salze, wird mit kaltem Wasser versetzt und unter gutem Rühren auf Temperaturen von etwa − 10 bis + 30 °C, vorzugsweise auf Temperaturen von − 8 bis + 5 °C abgekühlt. Die zugegebene Wassermenge soll das 0,5 bis 3 fache der zu erwartenden Diacetonitrilmenge betragen, vorzugsweise etwa das 2 fache. Dabei kristallisiert Diacetonitril in reinen, grobkörnigen Kristallen aus, und wird durch Filtration oder Zentrifugation abgetrennt und getrocknet. Erstaunlicherweise enthält das zentrifugenfeuchte, grobkristalline Produkt nur einige % Wasser, durch gelindes Trocknen im Vakuum bei 35-40 °C sinkt der Wassergehalt rasch auf unter 0,2 %.

Die Zentrifugenmutterlauge, die wegen der gewissen Wasserlöslichkeit des Diacetonitrils etwas Diacetonitril enthält, wird bevorzugterweise zur Zersetzung des im ersten Reaktionsschritt gebildeten Natriumsalzes von Diacetonitril verwendet. Hiedurch wird die Reinheit des Produktes nicht beeinträchtigt.

Das solcherart hergestellte Produkt ist äußerst rein, der Diacetonitrilgehalt ist über 99,5 %, es braucht nicht umkristallisiert zu werden.

Der Gehalt an 4-Amino-2,6-dimethyl-pyrimidin (Kyanmethin) beträgt unter 0,1 % der Gehalt an NaCN unter 0,05 % und 5-Amino-4-cyan-3-methyl-2,4-hexadiennitril konnte nicht nachgewiesen werden.

Beispiel 1

a) 92 g Natrium (4 Mole) werden mit 700 ml Benzin (Siedebereich 100-140 °C) in einem mit Rührer

ausgestatteten, mit Stickstoff inertisierten Reaktor eingeführt und auf 105 °C erwärmt. Sodann werden 265 g Acetonitril (6,5 Mole) kontinuierlich zugeführt, wobei die Reaktionstemperatur durch Wärmeabführung auf etwa 110 °C gehalten wird. Es wird im Reaktor ein Druck von 3-5 bar eingehalten und während der gesamten Reaktionszeit Methan abgeblasen. Die Acetonitrileintragung ist nach 20 Minuten beendet. Nach einer kurzen Nachreaktionszeit von 25 Minuten wird entspannt und das überschüssige Acetonitril abdestilliert.

Das Reaktionsgemisch wird auf 30-40 °C abgekühlt und 290 ml Wasser unter Rühren zugegeben. Die Temperatur wird durch Wärmeabfuhr konstant gehalten, die Eintragung ist nach 10 Minuten beendet. Sodann werden die drei dabei entstehenden Phasen getrennt. Die mittlere, das Diacetonitril enthaltende ölige Phase wird mit 200 ml Wasser versetzt und unter Rühren auf unter 0 °C abgekühlt. Nach dem Animpfen mit einigen Diacetonitrilkristallen kristallisiert das Produkt in groben, weißen bis hellgelben Kristallen, die unter Zentrifugation gewonnen und getrocknet werden.

| | |
|---|---|
| Ausbeute : | 81,7 % |
| Gehalt : | 99,5 % |
| 4-Amino-2,6-dimethylpyrimidin (aus GC) : | unter 0,1 % |
| NaCN : | unter 0,05 % |
| $H_2O$ : | ca. 0,2 % |
| 5-Amino-4-cyano-3-methyl-2,4-hexadiennitril (aus GC) : | nicht nachweisbar |

b) In einem zweiten Ansatz wird mit gleichen Mengen und unter gleichen Bedingungen gearbeitet, mit der Ausnahme, daß das rückgewonnene Acetonitril des ersten Ansatzes und das bei der Phasentrennung anfallende Benzin zur Reaktion mitverwendet werden. Ferner wird die beim Zentrifugieren des ersten Ansatzes anfallende Mutterlauge zum Zersetzen des Natriumsalzes herangezogen.

c) Vorgangsweise b) wird noch dreimal wiederholt und sodann bilanziert. Es werden pro Versuch im Durchschnitt 151 g Diacetonitril (kaum gefärbt, kristallin) mit einem Gehalt von 99,5 % erhalten ; das entspricht einer Ausbeute von 92,0 % der Theorie.

Beispiel 2

92 g Natrium werden in einen beheizbaren, mit Rührer versehenen, mit Stickstoff inertisierten Reaktor eingebracht, mit 1 300 ml Dekalin überschichtet und auf 100 °C gebracht. Nach Erreichen dieser Temperatur wird Acetonitril durch ein bis zum Boden des Reaktors reichendes Tauchrohr mittels einer Dosierpumpe eingebracht. Der nicht umgesetzte Acetonitrildampf wird mittels eines Kühlers kondensiert und über die Dosierpumpe in den Reaktor rückgeführt. Dieser Vorgang wird so lange weitergeführt, bis die theoretische Acetonitrilmenge von 247 g verbraucht wurde. Dies ist nach ca. 50 Minuten der Fall.

Nach Beendigung der Acetonitrilzufuhr wird die Suspension abgekühlt und sodann durch Zugabe von 300 ml Wasser unter Rühren bei ca. 30 °C hydrolysiert, nach Abtrennung der Natriumcyanid enthaltenden Wasserphase werden Dekalin- und Ölphase gemeinsam bei guter Rührung mit 100 ml Wasser gewaschen. Daraufhin wird die das Diacetonitril enthaltende Ölphase abgetrennt, in 200 ml Wasser eingerührt und nach Zusatz von einigen Diacetonitrilkristallen bei einer Temperatur unter 0 °C kristallisiert und zentrifugiert. Es werden 149 g Diacetonitril mit einem Gehalt von 99,5 % erhalten. Eine weitere Verbesserung der Ausbeute ist möglich, wenn das Waschwasser zum Zersetzen des Natriumsalzes des nächsten Ansatzes verwendet wird und die Zentrifugenmutterlauge anstelle reinen Wassers zum Kristallisieren darauffolgender Ansätze benützt wird.

Beispiel 3

16 kg Natrium werden mit 140 l Benzin (Kp 100-140 °C) in einem mit Stickstoff inertisierten Rührbehälter geschmolzen. Danach werden bei laufendem Rührer 47 kg Acetonitril (10 % Überschuß) bei einer Temperatur von 100-110 °C und unter einem Druck von 3-5 bar innerhalb von 40 Minuten eingetragen, wobei das gebildete Methan kontinuierlich abgeblasen, und die Reaktionswärme durch ständiges Kühlen abgeführt wird. Das überschüssige Acetonitril wird abgestrippt, die Suspension auf 30-40 °C abgekühlt und mit 52 l Wasser bei 30-40 °C innerhalb von 15 Minuten hydrolysiert. Aus dem entstandenen 3-Phasen-Gemisch werden die wäßrige, NaOH und NaCN enthaltende Schicht und die Benzin-Schicht abgetrennt, und die mittlere, das Diacetonitril enthaltende Ölschicht, bei einer Temperatur von ca.-4 bis 0 °C mit 35 l Wasser versetzt. Der weiße, grobkristalline Niederschlag von Diacetonitril wird abzentrifugiert, kurz nachgewaschen und im Vakuum bei 35-40 °C getrocknet.

Der Versuch wird 5 mal wiederholt, wobei das Benzin jeweils wieder zur Gänze eingesetzt wird, und ca. 90 % der Zentrifugenmutterlauge und das Waschwasser jeweils zum Zersetzen des Natriumsalzes von Diacetonitril verwendet werden. Die Ausbeute pro Versuch beträgt 26 kg, entsprechend 91 % der Theorie.

| | |
|---|---|
| Analyse : Diacetonitril : | 99,7 % |
| Kyanmethin aus GC : | unter 0,1 % |
| NaCN : | unter 0,05 % |

| | |
|---|---|
| $H_2O$ : | ca. 0,2 % |
| 5-Amino-4-cyano-3-methyl-2,4-hexadiennitril (aus GC) : | nicht nachweisbar. |

**Patentansprüche**

1. Verfahren zur Herstellung von reinem Diacetonitril durch Umsetzen von Acetonitril mit Natrium in einem inerten, unter Reaktionsbedingungen flüssigen organischen Medium, anschließende Hydrolyse des in erster Stufe gebildeten Natriumsalzes und Gewinnung des Diacetonitrils aus dem dabei entstehenden 3-Phasengemisch, dadurch gekennzeichnet, daß die Umsetzung mit geschmolzenem Natrium bei einer Temperatur von 97 bis 140 °C und unter einem Druck von 1 bis 10 bar durchgeführt wird, aus dem nach der Hydrolyse gebildeten Dreiphasensystem die wäßrige Phase und das inerte flüssige Medium abgetrennt werden, die dritte Phase aus rohem flüssigem Diacetonitril mit der 0,5- bis 3 fachen Gewichtsmenge Wasser, bezogen auf Gewicht Diacetonitril, versetzt und auf – 10 °C bis + 30 °C abgekühlt wird, worauf das reine Diacetonitril auskristallisiert und abgetrennt wird.

2. Verfahren nach Anspruch 1, dadurch gekennzeichnet, daß die Umsetzung bei einer Temperatur von 100-110 °C durchgeführt wird.

3. Verfahren nach den Ansprüchen 1 und 2, dadurch gekennzeichnet, daß die Umsetzung bei einem Druck von 2-5 bar durchgeführt wird.

4. Verfahren nach den Ansprüchen 1 bis 3, dadurch gekennzeichnet, daß die beim Auskristallisieren des Diacetonitrils entstehende Mutterlauge jeweils zur Hydrolyse des in erster Stufe gebildeten Natriumsalzes verwendet wird.

5. Verfahren nach den Ansprüchen 1-4, dadurch gekennzeichnet, daß das inerte flüssige organische Medium nach der Abtrennung für eine weiter Umsetzung mit Natrium verwendet wird.

6. Verfahren nach den Ansprüchen 1-5, dadurch gekennzeichnet, daß das Diacetonitril bei einer Temperatur von – 8 °C bis + 5 °C auskristallisiert wird.

**Claims**

1. Process for the preparation of pure diacetonitrile by reacting acetonitrile with sodium in an inert organic medium which is liquid under the reaction conditions, subsequently hydrolysing the sodium salt formed in the first stage and isolating the diacetonitrile from the 3-phase mixture thus produced, characterised in that the reaction is carried out with molten sodium at a temperature of 97 to 140 °C and under a pressure of 1 to 10 bar, the aqueous phase and the inert liquid medium are separated out from the three-phase system formed after hydrolysis, to the third phase, consisting of crude liquid diacetonitrile, is added from 0.5 to 3 parts by weight of water per part by weight of diacetonitrile and this mixture is cooled to between – 10 °C and + 30 °C, whereupon the pure diacetonitrile crystallises out and is separated off.

2. Process according to Claim 1, characterised in that the reaction is carried out at a temperature of 100-110 °C.

3. Process according to Claims 1 and 2, characterised in that the reaction is carried out at a pressure of 2-5 bar.

4. Process according to Claims 1 to 3, characterised in that the mother liquor formed when the diacetonitrile crystallises out is in each case used to hydrolyse the sodium salt formed in the first stage.

5. Process according to Claims 1-4, characterised in that the inert liquid organic medium, after having been separated off, is used for a further reaction with sodium.

6. Process according to Claims 1-5, characterised in that the diacetonitrile is crystallised out at a temperature of between – 8 °C and + 5 °C.

**Revendications**

1. Procédé pour la production de diacétonitrile pur par réaction d'acétonitrile avec du sodium dans un milieu organique inerte liquide dans la condition de réaction, puis hydrolyse du sel de sodium formé au cours du premier stade opératoire et récupération du diacétonitrile à partir du mélange en trois phases alors formé, caractérisé en ce qu'on effectue la réaction avec du sodium fondu à une température de 97 à 140 °C et sous une pression de 1 à 10 bars, on isole à partir du système en trois phases formé après l'hydrolyse la phase aqueuse et le milieu liquide inerte, on ajoute à la troisième phase, formée de diacétonitrile brut liquide, une quantité représentant de 0,5 à 3 fois la quantité en poids d'eau, sur la base du poids du diacétonitrile, et on refroidit entre – 10 °C et + 30 °C, après quoi le diacétonitrile pur est séparé par cristallisation et isolé.

2. Procédé suivant la revendication 1, caractérisé en ce qu'on effectue la réaction à une température de 100-110 °C.

3. Procédé suivant les revendications 1 et 2, caractérisé en ce qu'on effectue la réaction sous une pression de 2-5 bars.

4. Procédé suivant l'une quelconque des revendications 1 à 3, caractérisé en ce qu'on utilise la liqueur-mère obtenue lors de la séparation du diacétonitrile par cristallisation chaque fois pour l'hydrolyse du sel de sodium formé au cours du premier stade opératoire.

5. Procédé suivant l'une quelconque des revendications 1-4, caractérisé en ce qu'on utilise le milieu inerte organique liquide, après l'isolement, pour une nouvelle réaction avec du sodium.

6. Procédé suivant l'une quelconque des revendications 1-5, caractérisé en ce qu'on isole le diacétonitrile par cristallisation à une température de − 8 °C à + 5 °C.